(19) Europäisches Patentamt
European Patent Office
Office européen des brevets

(11) Veröffentlichungsnummer : **0 326 073 B1**

(12) # EUROPÄISCHE PATENTSCHRIFT

(45) Veröffentlichungstag der Patentschrift : **03.06.92 Patentblatt 92/23**

(51) Int. Cl.$^5$ : **C07J 41/00,** C07H 15/203, G01N 33/74, G01N 33/531

(21) Anmeldenummer : **89101185.0**

(22) Anmeldetag : **24.01.89**

(54) **Hapten-Protein-Konjugate und ihre Verwendung.**

(30) Priorität : **25.01.88 DE 3802060**

(43) Veröffentlichungstag der Anmeldung : **02.08.89 Patentblatt 89/31**

(45) Bekanntmachung des Hinweises auf die Patenterteilung : **03.06.92 Patentblatt 92/23**

(84) Benannte Vertragsstaaten : **AT BE CH DE ES FR GB GR IT LI LU NL SE**

(56) Entgegenhaltungen :
**EP-A- 0 218 347**
**FR-A- 2 262 670**
**GB-A- 2 165 046**
**US-A- 4 194 048**

(73) Patentinhaber : **BOEHRINGER MANNHEIM GMBH
Patentabteilung, Abt. E Sandhofer Strasse 112-132 Postfach 31 01 20
W-6800 Mannheim 31 Waldhof (DE)**

(72) Erfinder : **Huber, Erasmus, Dr.rer.nat.
Bürgerplatz 18
W-8046 Garching (DE)**
Erfinder : **Batz, Hans-Georg, Dr.rer.nat.
Traubinger-Strasse 63
W-8132 Tutzing (DE)**
Erfinder : **Von der Eltz, Herbert, Dr.rer.nat.
In der Au 21
W-8120 Weilheim (DE)**
Erfinder : **Klein, Christian, Dr.rer.nat.
Blütenstrasse 16
W-8120 Weilheim (DE)**

(74) Vertreter : **Huber, Bernhard, Dipl.-Chem. et al
Patentanwälte H. Weickmann, Dr. K. Fincke F.A. Weickmann, B. Huber Dr. H. Liska, Dr. J. Prechtel Kopernikusstrasse 9 Postfach86 08 20
W-8000 München 86 (DE)**

**EP 0 326 073 B1**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft Hapten-Protein-Konjugate und ihre Verwendung.

In der klinischen Diagnostik werden in zunehmendem Maße Bestimmungsverfahren nach dem Prinzip des Immunoassays durchgeführt, die sich durch besondere Empfindlichkeit auszeichnen. Dazu wird die zu bestimmende Substanz mit einer spezifischen, immunologisch aktiven Substanz, die ein Antigen, ein Hapten oder ein Antikörper sein kann und die mit einer Markierung versehen ist, und meist noch mit mindestens einer weiteren spezifisch bindenden Substanz, die an eine Festphase gebunden ist, umgesetzt. Nach Trennung des an die Festphase gebundenen Konjugats aus spezifisch bindender Substanz, zu bestimmender Substanz und markierter, spezifisch bindender Substanz von der flüssigen Phase kann dann in einer der beiden Phasen die Menge an Markierung bestimmt werden, die ein Maß für die zu bestimmende Substanz ist. Als Markierung werden sehr häufig Enzyme verwendet.

Für diese Immunoassays gibt es sehr viele Verfahrensvarianten. Für viele Zwecke werden z.B. kompetitive Assays durchgeführt. Dabei konkurrieren die zu bestimmende Substanz und eine zu der Probe zugegebene bekannte Menge an zu bestimmender Substanz, die mit einem Enzym markiert ist, um einen Antikörper.

Es besteht daher einerseits ein Bedarf an Konjugaten aus einem Markierungsenzym und einer mit der zu bestimmenden Substanz bzw. einem Antikörper spezifisch bindefähigen Verbindung, wobei das Konjugat nur mit der zu bestimmenden Substanz bzw. dem Antikörper reagieren darf und keine Kreuzreaktivitäten mit anderen in der Probelösung vorhandenen Verbindungen aufweisen darf, um das Ergebnis nicht zu verfälschen. Da die spezifisch bindende Substanz und das Markierungsenzym häufig nicht direkt aneinander gebunden werden, sondern über einen Spacer gekuppelt werden, führt die Affinität der Antikörper zu dem Spacer zu Problemen.

Weiterhin besteht ein großer Bedarf an spezifischen Antikörpern. Im allgemeinen werden Antikörper hergestellt, indem man ein Immunogen wie ein Antigen oder Hapten in geeigneter Form mehrmals in einen zur Antikörperbildung befähigen Organismus injiziert. Haptene sind definitionsgemäß Moleküle mit einem Molekulargewicht von weniger als 1.000 Dalton, die allein nicht immunogen wirksam sind, jedoch durch Bindung an ein Protein immunogen werden. Zur Immunisierung wird das Hapten mit einem immunogenen Molekül wie z.B. einem Serumprotein konjugiert. Injiziert man ein derartiges Konjugat, so bildet der Organismus neben Anti-Protein-Antikörpern die gewünschten Anti-Hapten-Antikörper. Die gebildeten Antikörper werden dann aus dem Organismus gewonnen. Auf diese Weise können polyklonale Antikörper gewonnen werden.

Auch zur Herstellung monoklonaler Antikörper muß zuerst eine Immunisierung eines geeigneten Organismus, in der Regel Mäuse, durchgeführt werden. Durch die üblicherweise mehrmalige Injektion eines Antigens oder Hapten-Konjugats wird eine B-Zelle dazu veranlaßt, Antikörper gegen dieses Antigen oder Hapten zu synthetisieren und zu sezernieren. Durch Screening wird dann eine B-Zelle, die die gewünschten Antikörper produziert aus der Milz isoliert und durch Fusionierung mit einer Myelomzelle immortalisiert. Diese Zelle erzeugt dann ständig dieselben, monoklonalen Antikörper.

Bei den üblicherweise zur Immunisierung verwendeten Konjugaten, bei denen das Hapten über eine Brücke an das immunogene Protein gebunden ist, treten nun häufig Kreuzreaktionen mit dem Brückenmolekül auf und die erhaltenen Antikörper zeigen eine zum Teil recht hohe Affinität für die im Immunogen vorhandenen Brückenstrukturen. Diese Brückenerkennung führt in immunologischen Tests oft zu Problemen.

Es war daher Aufgabe der Erfindung, Hapten-Protein-Konjugate zur Verfügung zu stellen, die zur Bildung spezifisch gegen das Hapten gerichteter Antikörper führen und keine Affinität zu dem Brückenmolekül haben.

Weiterhin war es Aufgabe der Erfindung, Hapten-Protein-Konjugate zur Verfügung zu stellen, die in Immunoassays eingesetzt werden können und zu einer spezifischen Bindung von Antikörpern am Hapten führen, ohne daß Kreuzreaktivitäten mit dem Brückenmolekül auftreten.

Diese Aufgabe wird gelöst durch Hapten-Protein-Konjugate, die dadurch gekennzeichnet sind, daß ein Hapten an das reduzierende Ende eines Zuckers, der aus 1 bis 10 Pyranose- oder Furanoseeinheiten besteht, gebunden ist und daß an eine freie $CH_2OH$-Gruppe am anderen Ende des Zuckers, die sich alpha-ständig zu einer OH-Gruppe befindet, ein Protein gebunden ist.

Überraschenderweise gelingt es, durch Verwendung eines Hapten-Protein-Konjugats, bei dem das Hapten an das Protein über einen Zucker gebunden ist, Antikörper zu erhalten, die sehr wenig Kreuzreaktivität mit der Brückenverbindung aufweisen. Die erhaltenen Antikörper haben eine hohe Affinität zu dem Hapten. Bei Verwendung der erfindungsgemäßen Hapten-Protein-Konjugate zur Immunisierung werden hohe Antikörpertiter erhalten. Weiterhin sind die erfindungsgemäßen Hapten-Protein-Konjugate besonders geeignet zum Einsatz in Immunoassays. Da die Antikörper zu dem Brückenmolekül keine Affinität aufweisen und daher sehr spezifisch nur das Hapten binden, erhält man mit diesen Konjugate bei der Verwendung in Immunoassays genauere Ergebnisse.

Bei den erfindungsgemäßen Hapten-Protein-Konjugaten ist das Hapten mit einem Protein über einen

Zucker verbunden. Dieser als Brücke verwendete Zucker kann 1 bis 10 Monosaccharideinheiten aufweisen. Bevorzugt werden Zucker mit 1 bis 5 Monosaccharideinheiten verwendet. Die Monosaccharideinheitenkönnen gleich oder verschieden sein und stellen Pyranosen oder Furanosen dar. Wesentlich ist nur, daß der Zucker ein reduzierendes Ende aufweist und am anderen Ende eine freie $CH_2OH$-Gruppe mit einer dazu alpha-ständigen Hydroxylfunktion besitzt.

Bevorzugt werden als Zucker Verbindungen verwendet, die leicht erhältlich sind, wie Glucose, Maltose oder Maltopentaose. Geeignet sind aber ebenso Lactose, Cellobiose oder auch Amylopektineinheiten.

Der Zucker wird an das Hapten gebunden über das reduzierende Ende. Die Bindung erfolgt in an sich bekannter Weise über funktionelle Gruppen des Haptens. Wenn das Hapten eine OH-Gruppe aufweist, so erfolgt die Bindung zwischen der Lactolgruppe des Zuckers und der OH-Gruppe unter Acetalbildung. Weist das Hapten keine geeignete funktionelle Gruppe auf, so muß eine geeignete Gruppe eingeführt werden. Die Bindung des Haptens an den Zucker muß in jedem Fall so erfolgen, daß sein Epitop frei zugänglich bleibt.

Der Zucker wird üblicherweise vor der Umsetzung mit dem Hapten peracetyliert. Nach der Umsetzung mit dem Hapten werden die Schutzgruppen in an sich bekannter Weise abgespalten und dann die freie $CH_2OH$-Gruppe selektiv derivatisiert bzw. aktiviert.

Zur Bindung des Glucose-Hapten-Konjugates an ein geeignetes Protein wird die freie $CH_2OH$-Gruppe des Zuckers aktiviert. Dies erfolgt in an sich bekannter Weise. Zur Aktivierung des Zuckers können an sich bekannte Verbindungen verwendet werden. Geeignet sind beispielsweise N-Hydroxysuccinimidester.

Das aktivierte Glucose-Hapten-Konjugat wird dann unter Standardbedingungen an eine freie $\varepsilon$-Aminogruppe des gewünschten Proteins gekuppelt. Als Protein geeignet sind die üblicherweise verwendeten immunogenen Carrier-Proteine, wie z.B. Edestin oder Rinderserumalbumin oder die in Immunoassays verwendeten Enzyme. Bevorzugt werden als Enzyme $\beta$-Galactosidase und Peroxidase verwendet.

In einer besonders bevorzugten Ausführungsform der Erfindung werden Hapten-Protein-Konjugate zur Verfügung gestellt, die aus einem Steroidhormon, einer Zuckerbrücke und einem Immunogenprotein bestehen. Der Nachweis von Steroidhormonen wie Östrogenen, Testosteron, Cortisonen und Herzglucosiden, denen allen das Steroidgrundgerüst gemeinsam ist, ist für die Diagnostik sehr wichtig. Die Zurverfügungstellung von Antikörpern gegen Steroidhormone sowie von Steroidhormon-Enzym-Konjugaten für die Durchführung von Immunoassays ist daher wünschenswert. Es hat sich nun als sehr vorteilhaft erwiesen, Steroidhormone so zu derivatisieren, daß sie an dem $C_6$-Atom eine OH-Gruppe tragen. Diese OH-Gruppe kann dann mit der Lactolgruppe der Zuckerbrücke unter Acetalbildung in an sich bekannter Weise reagieren. Diese Bindung beeinträchtigt nicht das Epitop des Steroidhormons und führt zu keinen unerwünschten Veränderungen des Moleküls. Je nach Verwendungszweck kann dann an das Konjugat aus Steroidhormon und Zuckerbrücke entweder ein immunogenes Protein oder ein Enzym gekuppelt werden.

Die erfindungsgemäßen Hapten-Protein-Konjugate eignen sich zur Immunisierung. Bevorzugt wird bei der Verwendung für die Immunisierung ein Konjugat verwendet, bei dem das Protein ein immunogenes Carrier-Protein, wie z.B. Edestin oder Rinderserumalbumin, ist. Es sind jedoch auch Konjugate, die Enzyme als Protein enthalten, für die Immunisierung geeignet. Das erfindungsgemäße Konjugat wird in den zur Antikörperbildung geeigneten Organismus in Abständen mehrmals injiziert. Das Konjugat bewirkt dann die Bildung von Antikörpern, die in sehr hohem Prozentsatz nur gegen das Hapten gerichtet sind und keine Kreuzreaktivität mit der Zuckerbrücke aufweisen. Die Antikörper können dann in an sich bekannter Weise aus dem Organismus gewonnen werden. Die erfindungsgemäß verwendeten Hapten-Carrier-Konjugate eignen sich sowohl zur Immunisierung für die Herstellung polyklonaler Antikörper als auch zur Herstellung monoklonaler Antikörper.

In einer weiteren Ausführungsform der vorliegenden Erfindung wird ein Haptenproteinkonjugat aus einem Hapten, das über eine Zuckerbrücke an ein Enzym gebunden ist verwendet zur Durchführung von Immunoassays. Das so gebildete Hapten-Enzym-Konjugat kann dann als Markierungsenzym in Bestimmungsverfahren nach dem Prinzip des Immunoassays eingesetzt werden. Dabei wird dann das aus Hapten und Zuckerbrücke gebildete Konjugat über die aktivierte $CH_2OH$-Gruppe mit einer freien $NH_2$-Gruppe des Enzyms umgesetzt.

Das erfindungsgemäße Hapten-Enzym-Konjugat kann z.B. in bekannter Menge einer Probelösung zugesetzt werden und konkurriert dann um einen gegen das Hapten gerichteten Antikörper.

Als besonders vorteilhaft hat es sich erwiesen, für die Immunisierung und für die Durchführung von Immunoassays verschiedene Konjugat zu verwenden, die sich in der Art der Brücke unterscheiden. Wird für die Immunisierung eine der bisher bekannten Brückenverbindungen wie Carboxymethoxim, Dimethylcarboxymethoxim oder Hemisuccinat verwendet, so sollte das im Immunoassay verwendete Hapten-Enzym-Konjugat eine Zuckerbrücke aufweisen. Werden für den Immunoassay Antikörper verwendet, die durch Immunisierung mit einem erfindungsgemäßen Hapten-Protein-Konjugat mit Zuckerbrücke erhalten wurden, so hat es sich als vorteilhaft erwiesen, ein Hapten-Enzym-Konjugat mit bekannter, bevorzugt hydrophober Brücke einzusetzen.

Die Erfindung wird durch die folgenden Beispiele erläutert:

**Beispiel 1**

Vorschrift zur Herstellung von Oestradiol-6α-maltose-β-Gal-Immunogen

1. Oestradiol-3,17-diacetat (II)

600 ml Acetanhydrid werden unter Rühren und Kühlen mit 1,2 ml Perchlorsäure versetzt. Anschließend gibt man portionsweise 54,4 g (200 mmol) Oestradiol (I) bei 30 bis 40°C zu und läßt 30 min bei 25°C rühren. Das Acetanhydrid wird bei 40°C Wasserbadtemperatur im Hochvakuum abgedampft und der Rückstand in 1 l Diethylether gelöst. Man filtriert durch ein Faltenfilter und wäscht das Filtrat zweimal mit je 500 ml ges. $NaHCO_3$-Lösung. Die Etherphase wird abgetrennt, mit 500 ml Wasser gewaschen und mit 50 g $Na_2SO_4$ getrocknet. Das Lösungsmittel wird abgedampft und der Rückstand mit 500 ml Petrolether digeriert. Das feste Produkt wird abgesaugt und im Exsikkator über $CaCl_2$ getrocknet.

2. 6-Oxo-oestradiol-3,17-diacetat (III)

35,6 g (100 mmol) II werden in 500 ml Eisessig gelöst. Man läßt 142 g $CrO_3$ in 820 ml Eisessig und 110 ml Wasser gelöst bei 20°C zutropfen und rührt 2 Stunden bei 25°C. Dann gießt man in 12 l Wasser und extrahiert mit 5x500 ml Dichlormethan. Die vereinigten organischen Extrakte werden mit 1 l Wasser gewaschen, über 50 g $Na_2SO_4$ getrocknet und eingedampft.

Das Produkt III wird aus dem erhaltenen zähflüssigen Rückstand (38g) durch präparative Säulenchromatographie an Kieselgel (Eluent: Essigester/Petrolether 1:1) abgetrennt.
Ausbeute: 12,5 g (34% zähes Öl).

3. 6α-Hydroxy-oestradiol-3,17-diacetat (IV)

9,3 g (25 mmol) III werden in 300 ml wasserfreiem Ethanol und 150 ml wasserfreiem Tetrahydrofuran gelöst, auf 0°C abgekühlt und unter Rühren mit 3,8 g (100 mmol) $NaBH_4$ versetzt. Nach 1,5 Stunden bei 0°C verdünnt man mit 1 l Diethylether, wäscht zweimal mit je 500 ml Wasser und trocknet über 30 g $Na_2SO_4$. Das Lösungsmittel wird abgedampft und der Rückstand durch präparative Säulenchromatographie an Kieselgel (Eluent: Essigester/Petrolether 1:1) aufgetrennt. Das Produkt IV wird als zähflüssiges, langsam kristallisierendes Öl erhalten.
Ausbeute: 4,2 g (45%).

4. Oestradiol-6α-maltosid-peracetat V

3,7 g (10 mmol) IV werden zusammen mit 11,7 g (15 mmol) Heptaacetylmaltosetrichloracetimidat (Herstellung analog R.R. Schmidt und J. Michel, Angew. Chem. 92 (1980), 763) in 100 ml wasserfreiem Dichlormethan gelöst. Unter Rühren werden 1,5 ml Bortrifluorid-Etherat in 5 ml Dichlormethan bei 20°C zugetropft. Nach 1,5 Stunden Rühren gibt man nochmals 0,1 ml Bortrifluorid-Etherat in 5 ml Dichlormethan zu und läßt weitere 15 Minuten rühren. Dann wäscht man dreimal mit je 50 ml ges. $NaHCO_3$-Lösung und anschließend mit 100 ml Wasser. Die organische Phase wird mit 10 g $Na_2SO_4$ getrocknet und eingedampft. Das Produkt V wird durch präparative Säulenchromatographie an Kieselgel (Eluent:Essigester/Petrolether 3:2) aus dem Rückstand abgetrennt.
Ausbeute: 1,3 g (31%, zähes Öl).

5. Oestradiol-6α-maltosid VI

0,99 g (1 mmol) V werden in 15 ml Methanol gelöst und mit 5 ml 2n NaOH versetzt. Man rührt 20 Stunden bei 25°C, dann wird der pH-Wert durch Zugabe von 2n HCl auf 5,0 gestellt und die Lösung im Vakuum zur Trockne eingedampft. Den Rückstand löst man in 10 ml Dimethylformamid, filtriert und dampft das Filtrat im Hochvakuum zur Trockne ein. Der Rückstand wird mit 10 ml Aceton digeriert, abgesaugt und getrocknet. Das Rohprodukt reinigt man durch präparative Säulenchromatographie an Kieselgel (Eluent: Chloroform/Methanol 3:2).
Ausbeute: 360 mg (52%).

6. Oestradiol-6α-maltose-hemiglutaryl-N-hydroxysuccinimidester VII

310 mg (0,5 mmol) VI werden in 10 ml wasserfreiem Dimethylformamid gelöst und mit 1,75 g (6 mmol) Glutarsäure-ω-o-trimethylester-ω'-N-hydroxysuccinimidester versetzt. Nach Zugabe von 300 mg Nafion 117 und 2 g Molekularsieb 4A läßt man 20 Stunden unter Inertgasatmosphäre rühren. Danach filtriert man über ein Faltenfilter, versetzt das Filtrat mit 2 ml 0,1 n HCl und rührt die Lösung 15 Minuten. Dann wird der pH-Wert mit 0,1 n NaOH auf 5,0 gestellt und die Lösung im Hochvakuum zur Trockne eingedampft. Den Rückstand löst man nochmals in 10 ml wasserfreiem Dimethylformamid, filtriert und dampft wiederum zur Trockne ein. Der Rückstand wird mit 10 ml wasserfreiem Essigester digeriert, dan gibt man 10 ml Petrolether zu und saugt den Feststoff ab. Das Produkt VII wird mit Diethylether gewaschen und im Hochvakuum bei 30°C getrocknet. Ausbeute: 280 mg (68%).

7. Oestradiol-Maltose-Immunogen VIII

100 mg aktiviertes Hapten VIII wird in 5 ml Dimethylformamid gelöst und zu einer Lösung von 1 g β-Galactosidase in 0,5 l 0,1 n Phosphatpuffer pH 8,5 gegeben. Man rührt 20 Stunden bei 25°C, dialysiert gegen Wasser und lyophilisiert. Das Lyophilisat wird zur Immunisierung verwendet.

Das Reaktionsschema ist der folgenden Darstellung zu entnehmen:

Reaktionsschema zu Beispiel 1

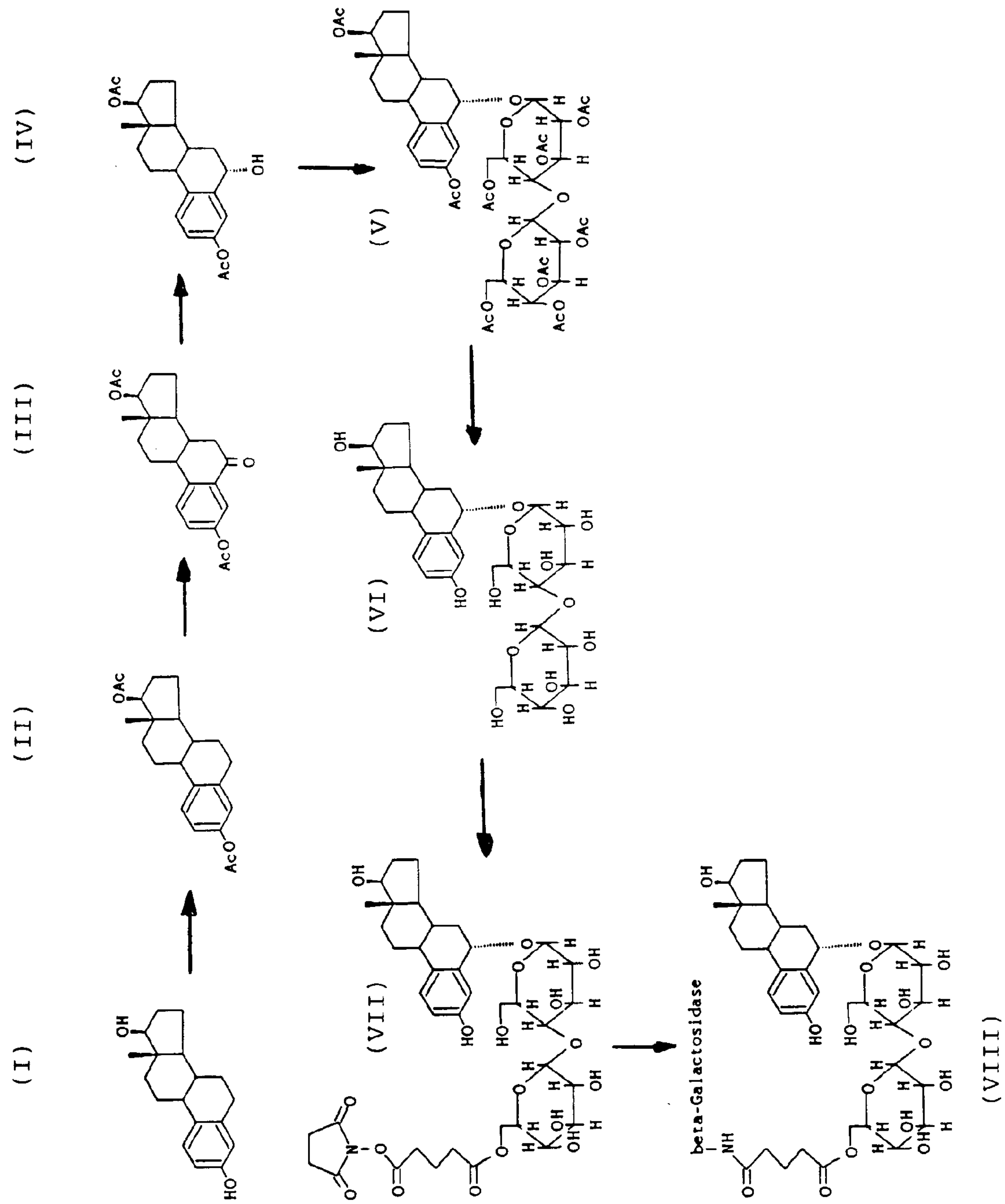

(I) (II) (III) (IV) (V) (VI) (VII) (VIII)

**Beispiel 2**

a) Diphenylhydantoin-4′-tetraacetylglucosid II

1,07 g (8 mmol) 5-(4-Hydroxyphenyl)-5-phenylhydantoin I werden zusammen mit 2,36 g (4,8 mmol) Tetraacetylglucose-trichloracetimidat (Herstellung analog R.R. Schmidt und J. Michel, Angew. Chem. 92 (1982), 763) in 40 ml wasserfreiem Tetrahydrofuran gelöst. Unter Rühren werden 0,15 ml Bortrifluorid-Etherat in 5 ml wasserfreiem Tetrahydrofuran bei 20°C zugetropft. Nach 16 Stunden Rühren bei Raumtemperatur gibt man nochmals 0,98 g (2 mmol) Tetraacetylglucose-trichloracetimidat und 0,1 ml Bortrifluorid-Etherat zu und läßt weitere 4 Stunden rühren. Danach versetzt man die Reaktionsmischung mit 2 g $NaHCO_3$, filtriert und dampft das Filtrat im Vakuum ein. Der Rückstand wird mit 20 ml Aceton extrahiert, der Extrakt im Vakuum eingedampft und das Produkt durch präparative Säulenchromatographie an Kieselgel (Eluent: Essigester/Petrolether 2:1) abgetrennt.
Ausbeute: 1,38 g (52%).

b) Diphenylhydantoin-4′-glucosid III

1,2 g (2 mmol) II werden in 15 ml Methanol gelöst und mit 100 mg Natriumethylat versetzt. Man läßt 20 Stunden bei 25°C rühren, dann gibt man 1 g Amberlite LG 50 $H^+$ zu und läßt weitere 15 Minuten rühren. Die Reaktionsmischung wird filtriert und das Filtrat im Vakuum eingedampft. Das Rohprodukt wird in wenig Methanol gelöst, auf eine Kieselgelsäule aufgetragen und mit Chloroform/Methanol 3:1 eluiert.
Ausbeute: 520 mg (61%).

c) Diphenylhydantoin-4′-glucose-hemiglutaryl-N-hydroxysuccinimidester IV

0,43 g (1 mmol) III werden in 10 ml wasserfreiem Tetrahydrofuran gelöst und mit 0,87 g (3 mmol) Glutarsäure-ω-o-trimethylester-ω′-N-hydroxysuccinimidester versetzt. Zu der Lösung gibt man 40 mg p-Toluolsulfonsäure und läßt 20 Stunden bei 25°C rühren. Anschließend wird die Lösung im Vakuum eingedampft, der Rückstand in 30 ml Essigester gelöst und mit je 20 ml 0,1 n HCl und Wasser gewaschen. Die organische Phase wird mit 5 g $Na_2SO_4$ getrocknet und im Vakuum eingedampft. Das Produkt IV trennt man durch präparative Säulenchromatographie an Kieselgel (Eluent: Essigester/Tetrahydrofuran 3:1) aus der Mischung ab.
Ausbeute: 0,26 g (41%).

d) Diphenylhydantoin-Glucose-Immunogen V

50 mg aktiviertes Hapten V werden in 5 ml Dimethylformamid gelöst und zu einer Lösung von 1 g RSA in 50 ml 0,1 n Phosphatpuffer pH 8,5 gegeben. Man rührt 5 Stunden bei 25°C, dialysiert gegen Wasser und lyophilisiert. Das Lyophilisat wird mit Aceton gewaschen, im Vakuum getrocknet und zur Immunisierung verwendet.

Das Reaktionsschema ist der folgenden Darstellung zu entnehmen:

Reaktionsschema zu Beispiel 2

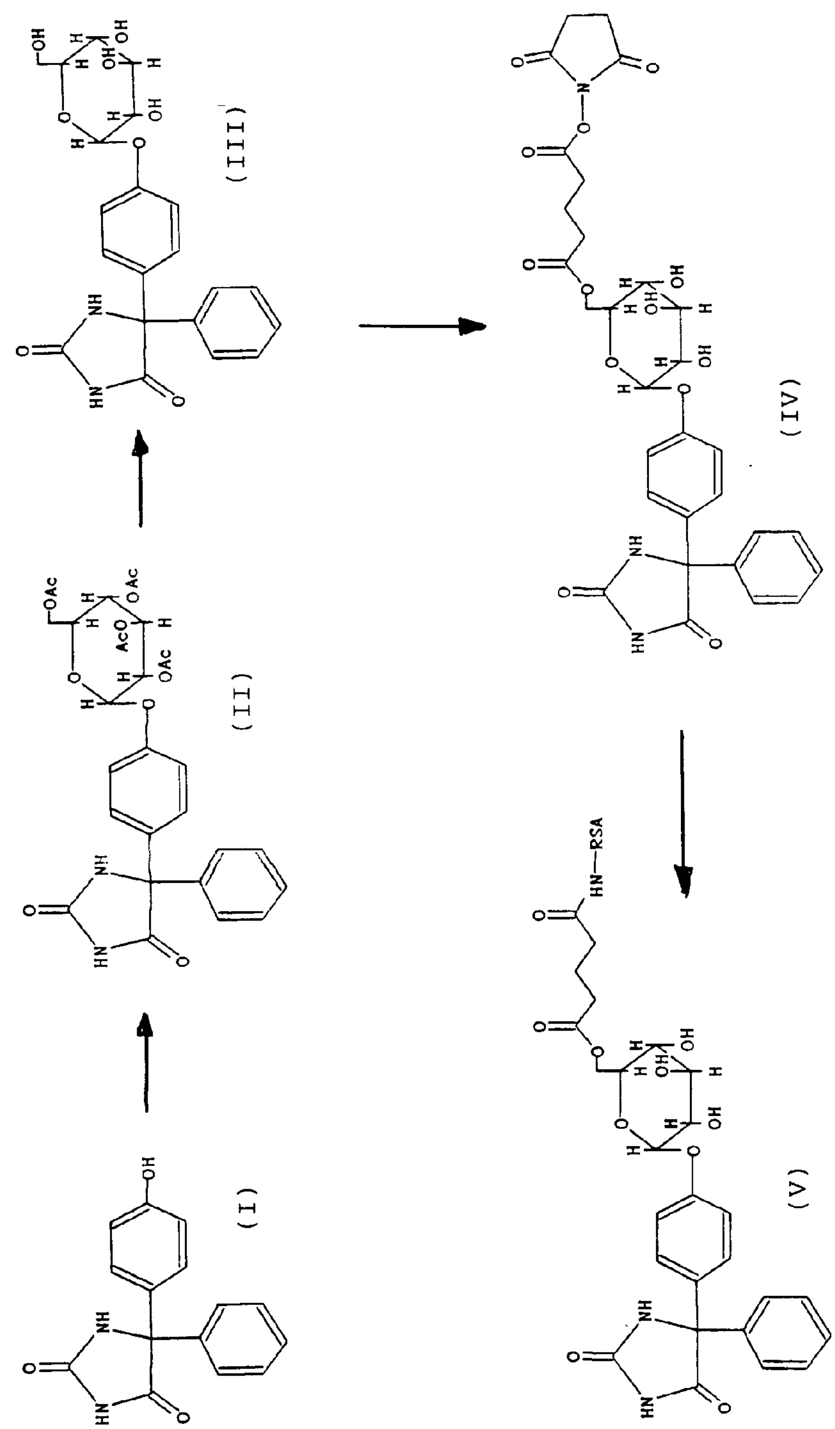

(I)

(II)

(III)

(IV)

(V)

**Beispiel 3**

Es wurden Antiseren durch Immunisierung mit erfindungsgemäßen Immunogenen und Immunogenen des Standes der Technik hergestellt und ihre Kreuzreaktivitäten verglichen.

Dazu wurden je 10 Schafe mit 100 µg Immunogen in Freud'schem Adjuvants erst immunisiert und in ca. 8-wöchigem Rhythmus mit je 50 µg Immunogen dreimal weiterimmunisiert. Nach 60 bzw. 150 Tagen wurde Antiserum abgenommen und immunsorptiv gereinigt.

Als Immunogen wurden Oestradiol-Proteinkonjugate verwendet. Als Trägerprotein wurde in allen Fällen β-Galactosidase verwendet. Für die Serien 1 -3 erfolgte die Bindung an Oestradiol nach dem Stand der Technik mit Carboxymethoxim (cmo), Dimethylcarboxymethoxim (dmc) und Hemisuccinat (hs) (s. Steroid Biochem. 22 (1985) 285, Chem. Pharm. Bull. 22 (1974) 1167, Steroids 23 (1974) 549). Die Formeln für die Verbindungen 1 bis 3 sind der folgenden Darstellung zu entnehmen.

cmo/dmc/hs-ε-Lysin-modifizierte Oestradiole (Beispiel 3)

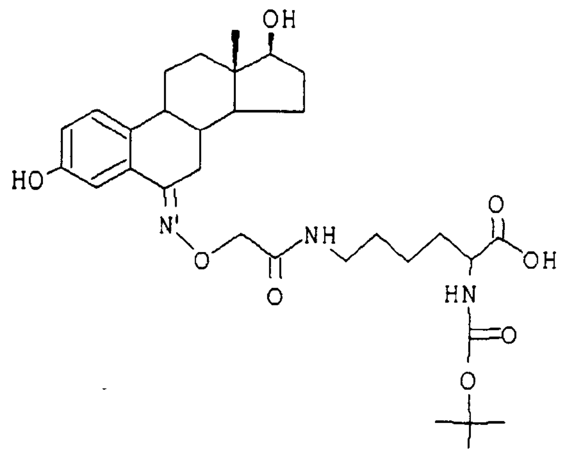

(1)

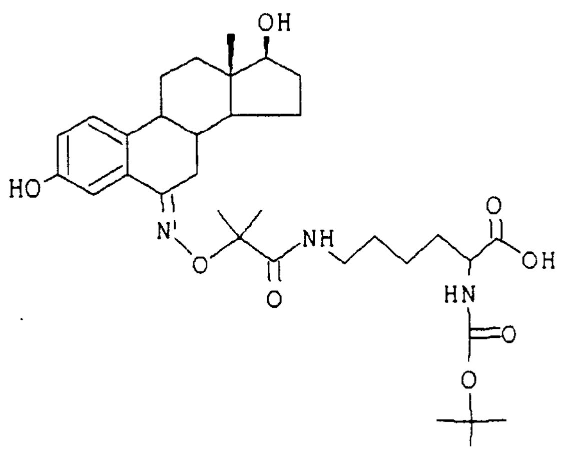

(2)

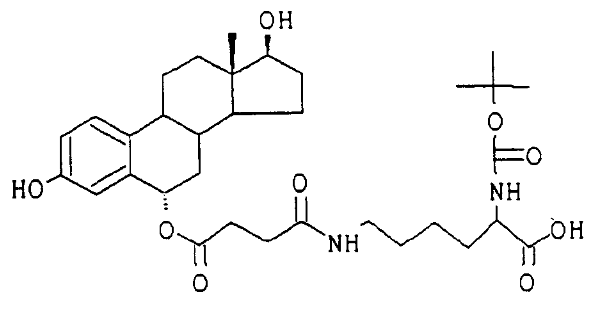

(3)

Bei drei weiteren Serien (4 - 6) wurde ein erfindungsgemäßes Immunogen mit Glucose als Brückenbildner verwendet. Die Herstellung erfolgte analog Beispiel 2.

Bestimmung der Kreuzreaktivität

1. Reagenzien

Beschichtungspuffer:

50 mmol/l Natriumcarbonat pH 9,6

Probenpuffer:

10 mmol/l Natriumphosphat pH 7,4 0,9 % NaCl, 0,1 % Tween 20, 1 % Crotein C.

Waschpuffer:

0,9 % NaCl, 0,1 % Tween 20

Antikörper-Enzym-Konjugat:

25 mU/ml Konjugat aus Peroxidase und einem polyklonalen Antikörper aus Kaninchen, der gegen Schaf Fc γ gerichtet ist, gelöst in Probenpuffer.

Substrat:

1,9 mmol/l ABTS®
(2,3′-Azino-di-[3-Ethyl-benzthiazolin-Sulfonsäure(6)]-Diammoniumsalz

Polyhapten:

Oestradiol an Kaninchen IgG, hergestellt analog Beispiel 1.7.

In der Serien 1 - 3 wurde ein Polyhapten analog Beispiel 1.7 verwendet. In den Serien 4 - 6 wurde ein Polyhapten verwendet, welches analog Beispiel 1.7. hergestellt wurde, wobei jedoch als aktiviertes Hapten Oestradiol-6-cmo-N-Hydroxysuccinimidester eingesetzt wurde.

Oestradiol-Derivate: Oestradiol-6-cmo-ε-Lysin (1)
Oestradiol-6-dmc-ε-Lysin (2)
Oestradiol-6-hs-ε-Lysin (3)

6-Glucosyl-Oestradiol (Herstellung analog Beispiel 1.5).

Die Herstellung der cmo/dmc/hs-ε-Lysin modifizierten Oestradiole erfolgte dadurch, daß 1 mmol/l Oestradiol-6-cmo/dmc/hs-N-Hydroxysuccinimidester und 1 mmol/l N-α-tert.-Butyloxycarbonyl-Lysin in 5 ml Dimethylformamid gelöst und 2 Tage bei Raumtemperatur inkubiert wurden. Das Produkt wurde durch Zutropfen von Diisopropyläther ausgefällt und im Vakuum getrocknet.

Antikörpertitration

In einem Vorversuch wurden die in die eigentlichen Spezifitätsexperimente einzusetzenden Antikörper-Mengen bestimmt. Dazu wurden die Microtiterplatten mit 100 µl/well einer Lösung von 1 µg Polyhapten/ml Beschichtungspuffer 1 Stunde unter Schütteln bei Raumtemperatur inkubiert. Anschließend wurde 3 x mit Waschpuffer gewaschen.

100 µl des Antiserums (Verdünnungsreihe ab 1 : 100 4er Schritte in Probenpuffer) wurden pro well zugegeben und 1 Stunde bei Raumtemperatur geschüttelt. Anschließend wurde 3 x mit Waschpuffer gewaschen.

Es wurden 100 µl Antikörper-Enzym-Konjugat zugegeben, eine Stunde bei Raumtemperatur geschüttelt und 3 x mit Waschpuffer gewaschen.

Die Nachweisreaktion wurde durch Zugabe von 100 µl Substrat/well gestartet. Nach 15 Minuten bei Raumtemperatur wurde bei 405 nm (Referenz-Wellenlänge 490 nm) gemessen.

Die zur halbmaximalen Bindung gehörige Antiserumverdünnung wurde als Titer definiert. Diese Antikörper-Menge wurde in die folgenden Experimente eingesetzt.

Spezifität des Antiserums (Kreuzreaktion)

Zur Untersuchung der Spezifität des Antiserums wurden die Reaktivitäten mit den in Lösung angebotenen Komponenten miteinander verglichen.

Die Mikrotiterplatten (A) wurden mit 100 µl/well einer Lösung von 1 µg/Polyhapten/ml in Beschichtungspuffer 1 Stunde bei Raumtemperatur inkubiert und 3 x mit Waschpuffer gewaschen.

Antiserum (in doppelter Titerkonzentration) und Antigen (Verdünnungsreihe ab 5 µg Oestradiol-Derivate/ml Probenpuffer (mit Probenpuffer in 4er Schritten) wurden in mit 1 % Crotein C vorbeschichtete Mikrotiterplatten (B) gemischt (50 µl + 50 µl) und 30 Minuten bei Raumtemperatur inkubiert. Die 100 µl Aliquots der Gemische wurden in die mit Polyhapten beschichteten Mikrotiterplatten (A) transferiert. Es wurde 1 Stunde bei Raumtemperatur unter Schütteln inkubiert und 3 x mit Waschpuffer gewaschen.

Es wurden 100 µl Antikörper-Enzym-Konjugat/well zugegeben und 1 Stunde bei Raumtemperatur geschüttelt. Anschließend wurde 3 x mit Waschpuffer gewaschen.

Die Nachweisreaktion wurde durch Zugabe von 100 µl Substrat/well gestartet und bei 405 nm (Referenz-Wellenlänge 490 nm) nach 15 Minuten bei Raumtemperatur gemessen.

Die zur halbmaximalen Bindung gehörige Konzentration eines Antigens wird als relative Affinität definiert.

Zum Vergleich der Reaktivität eines Antiserums mit verschiedenen Oestradiolderivaten wird die relative Affinität von Oestradiol = 100 % gesetzt. Die Reaktivitäten (entsprechend den Kreuzreaktionen) ergeben sich aus den Prozenten der relativen Affinität:

$$\text{Hreuzreaktion} = \frac{c_{\text{rel.Aff.Oestradiol (nM)}}}{c_{\text{rel.Aff.x (nM)}}} \times 100\ \%$$

Die Ergebnisse sind in Tabelle I angegeben. Hierbei zeigt sich deutlich die Überlegenheit der Immunogene mit Zuckerbrücke in der wesentlich niedrigeren Kreuzreaktion zum homologen Oestradiol-Derivat, die wiederum ein quantitativer Ausdruck für die gegen den Linker selbst gerichteten Antikörper ist. Je niedriger die Kreuzreaktion ist, desto besser ist das Ergebnis.

Tabelle I

| Serie | homologes Oestradiol-Derivat | Kreuzreaktion mit dem homologen Derivat (%) Probe 1 | Probe 2 |
|---|---|---|---|
| 1 | Oestradiol-6-cmo-ε-Lysin | 1040 | 770 |
| 2 | Oestradiol-6-dmc-ε-Lysin | 770 | 180 |
| 3 | Oestradiol-6-hs-ε-Lysin | 1950 | 620 |
| 4 | 6-Glucosyl-Oestradiol | 191 | 119 |
| 5 | 6-Glucosyl-Oestradiol | 163 | 121 |
| 6 | 6-Glucosyl-Oestradiol | 373 | 244 |

Probe 1: Serumentnahme 60 Tage nach Erstimmunisierung
Probe 2: Serumentnahme 150 Tage nach Erstimmunisierung

## Patentansprüche

### Patentansprüche für folgende Vertragsstaaten : At, BE, CH, LI, DE, FR, GB, GR, IT, LU, NL, SE

1. Hapten-Protein-Konjugat, **dadurch gekennzeichnet**, daß ein Hapten an das reduzierende Ende eines Zuckers, der aus 1 bis 10 Pyranose- oder Furanoseeinheiten besteht, gebunden ist und daß an eine freie $CH_2OH$-Gruppe am anderen Ende des Zuckers, die sich alpha-ständig zu einer OH-Gruppe befindet, ein Protein gebunden ist.

2. Hapten-Protein-Konjugat nach Anspruch 1, **dadurch gekennzeichnet,** daß man als Zucker Lactose, Cellobiose, Maltose oder Maltopentaose verwendet.

3. Hapten-Protein-Konjugat nach einem der Ansprüche 1 oder 2 **dadurch gekennzeichnet,** daß das Hapten ein Steroidhormon ist, das am $C_6$-Atom eine OH-Gruppe aufweist, über die ein Zucker mit seinem reduzierenden Ende gebunden ist.

4. Hapten-Protein-Konjugat nach Anspruch 3, **dadurch gekennzeichnet,** daß das Hapten Östradiol oder Digoxin ist.

5. Hapten-Protein-Konjugat nach einem der Ansprüche 1 bis 4, **dadurch gekennzeichnet,** daß das Protein ein Enzym ist.

6. Hapten-Protein-Konjugat nach Anspruch 5, **dadurch gekennzeichnet,** daß das Enzym β-Galactosidase oder Peroxidase ist.

7. Hapten-Protein-Konjugat nach einem der Ansprüche 1 bis 4, **dadurch gekennzeichnet,** daß an die freie, zu einer OH-Gruppe alpha-ständige $CH_2OH$-Gruppe ein immunogenes Protein gebunden ist.

8. Verwendung eines Hapten-Protein-Konjugats nach einem der Ansprüche 1 bis 7 zur Immunisierung von zur Antikörperbildung geeigneten Organismen.

9. Verwendung eines Hapten-Protein-Konjugats nach Anspruch 5 oder 6 als Markierungsenzym in einem Immunoassay.

### Patentansprüche für folgenden Vertragsstaat : ES

1. Verfahren zur Herstellung eines Hapten-Protein-Konju gats, **dadurch gekennzeichnet**, daß man an das reduzierende Ende eines Zuckers, der aus 1 bis 10 Pyranose- oder Furanoseeinheiten besteht, ein Hapten bindet und daß an eine freie $CH_2OH$-Gruppe am anderen Ende des Zuckers, die sich alpha-ständig zu einer OH-Gruppe befindet, ein Protein bindet.

2. Verfahren nach Anspruch 1, **dadurch gekennzeichnet**, daß man als Zucker Lactose, Cellobiose, Maltose oder Maltopentaose verwendet.

3. Verfahren nach einem der Ansprüche 1 oder 2, **dadurch gekennzeichnet**, daß man als Hapten ein Steroidhormon verwendet, das am $C_6$-Atom eine OH-Gruppe aufweist, über die ein Zucker mit seinem reduzierenden Ende gebunden ist.

4. Verfahren nach Anspruch 3, **dadurch gekennzeichnet**, daß man als Hapten Östradiol oder Digoxin verwendet.

5. Verfahren nach einem der Ansprüche 1 bis 4, **dadurch gekennzeichnet**, daß man als Protein ein Enzym verwendet.

6. Verfahren nach Anspruch 5, **dadurch gekennzeichnet**, daß man als Enzym β-Galactosidase oder Peroxidase ver wendet.

7. Verfahren nach einem der Ansprüche 1 bis 4, **dadurch gekennzeichnet**, daß man an die freie, zu einer OH-Gruppe alpha-ständige $CH_2OH$-Gruppe ein immunogenes Protein bindet.

8. Verwendung eines nach einem der Ansprüche 1 bis 7 hergestellten Hapten-Protein-Konjugats zur Immunisierung von zur Antikörperbildung geeigneten Organismen.

9. Verwendung eines nach Anspruch 5 oder 6 hergestellten Hapten-Protein-Konjugats als Markierungsenzym in einem Immunoassay.

## Claims

### Claims for the following Contracting States : AT, BE, CH, DE, FR, GB, GR, IT, LI, LU, NL, SE

1. Hapten-protein conjugate,
characterised in that
a hapten is bound to the reducing end of a sugar which consists of 1 to 10 pyranose or furanose units and that,

on a free $CH_2OH$ group on the other end of the sugar, which is present in the alpha-position to an OH group, is bound a protein.

2. Hapten-protein conjugate according to claim 1,
characterised in that one uses lactose, cellobiose, maltose or maltopentaose as sugar.

3. Hapten-protein conjugate according to one of claims 1 or 2,
characterised in that
the hapten is a steroid hormone which has an OH group on the $C_6$-atom via which a sugar is bound with its reducing end.

4. Hapten-protein conjugate according to claim 3,
characterised in that the hapten is oestradiol or digoxin.

5. Hapten-protein conjugate according to one of claims 1 to 5,
characterised in that
the protein is an enzyme.

6. Hapten-protein conjugate according to claim 5,
characterised in that the enzyme is β-galactosidase or peroxidase.

7. Hapten-protein conjugate according to one of claims 1 to 5,
characterised in that
an immunogenic protein is bound to the free $CH_2OH$ group present in the alpha-position to an OH group.

8. Use of a hapten-protein conjugate according to one of claims 1 to 7 for the immunisation of organisms suitable for the antibody formation.

9. Use of a hapten-protein conjugate according to claim 5 or 6 as labelling enzyme in an immunoassay.

**Claims for the following Contracting State : ES**

1. Process for the preparation of a hapten-protein conjugate,
characterised in that
one binds a hapten to the reducing end of a sugar which consists of 1 to 10 pyranose or furanose units and on a free $CH_2OH$ group on the other end of the sugar, which is present in the alpha-position to an OH group, binds a protein.

2. Process according to claim 1,
characterised in that
one uses lactose, cellobiose, maltose or malto-pentaose as sugar.

3. Process according to claim 1 or 2,
characterised in that
one uses a steroid hormone as hapten which has an OH group on the $C_6$-atom via which a sugar is bound with its reducing end.

4. Process according to claim 3,
characterised in that
one uses oestradiol or digoxin as hapten.

5. Process according to claims 1 to 4,
characterised in that
one uses an enzyme as protein.

6. Process according to claim 5,
characterised in that
one uses β-galactosidase or peroxidase as enzyme.

7. Process according to one of claims 1 to 4,
characterised in that
one binds an immunogenic protein to the free $CH_2OH$ group present in the alpha-position to an OH group.

8. Use of a hapten-protein conjugate prepared according to one of claims 1 to 7 for the immunisation of organisms suitable for the antibody formation.

9. Use of a hapten-protein conjugate prepared according to claim 5 or 6 as labelling enzyme in an immunoassay.

## Revendications

### Revendications pour les Etats contractants suivants : AT, BE, CH, DE, FR, GB, GR, IT, LI, LU, NL, SE

1. Conjugué haptène-protéine,
caractérisé en ce qu'un haptène est fixé à l'extrémité réductrice d'un sucre qui consiste en 1 à 10 unités pyranose ou furanose et en ce qu'une protéine est fixée à un groupe $CH_2OH$ libre situé en alpha d'un groupe OH à l'autre extrémité du sucre.

2. Conjugué haptène-protéine selon la revendication 1,
caractérisé en ce que l'on utilise comme sucre le lactose, le cellobiose, le maltose ou le maltopentaose.

3. Conjugué haptène-protéine selon l'une des revendications 1 ou 2,
caractérisé en ce que l'haptène est une hormone stéroïde qui présente sur l'atome $C_6$ un groupe OH par l'intermédiaire duquel un sucre est fixé par son extrémité réductrice.

4. Conjugué haptène-protéine selon la revendication 3,
caractérisé en ce que l'haptène est l'oestradiol ou la digoxine.

5. Conjugué haptène-protéine selon l'une des revendications 1 à 4,
caractérisé en ce que la protéine est une enzyme.

6. Conjugué haptène-protéine selon la revendication 5,
caractérisé en ce que l'enzyme est la β-galactosidase ou la peroxydase.

7. Conjugué haptène-protéine selon l'une des revendications 1 à 4,
caractérisé en ce qu'une protéine immunogène est fixée au groupe $CH_2OH$ libre situé en alpha d'un groupe OH.

8. Utilisation d'un conjugué haptène-protéine selon l'une des revendications 1 à 7 pour l'immunisation d'organismes convenant pour la formation d'anticorps.

9. Utilisation d'un conjugué haptène-protéine selon la revendication 5 ou 6 comme enzyme de marquage dans une détermination immunologique.

### Revendications pour l'Etat contractant suivant : ES

1. Procédé de préparation d'un conjugué haptène-protéine,
caractérisé en ce que
l'on fixe un haptène à l'extrémité réductrice d'un sucre qui consiste en 1 à 10 unités pyranose ou furanose et en ce que l'on fixe une protéine à un groupe $CH_2OH$ libre situé en alpha d'un groupe OH à l'autre extrémité du sucre.

2. Procédé selon la revendication 1,
caractérisé en ce que
l'on utilise comme sucre le lactose, le cellobiose, le maltose ou le maltopentaose.

3. Procédé selon l'une des revendications 1 ou 2,
caractérisé en ce que
l'on utilise comme haptène une hormone stéroïde qui présente sur l'atome $C_6$ un groupe OH par l'intermédiaire duquel un sucre est fixé par son extrémité réductrice.

4. Procédé selon la revendication 3,
caractérisé en ce que
l'on utilise comme haptène l'oestradiol ou la digoxine.

5. Procédé selon l'une des revendications 1 à 4,
caractérisé en ce que
l'on utilise une enzyme comme protéine.

6. Procédé selon la revendication 5,
caractérisé en ce que
l'on utilise comme enzyme la β-galactosidase ou la peroxydase.

7. Procédé selon l'une des revendications 1 à 4,
caractérisé en ce que
l'on fixe une protéine immunogène au groupe $CH_2OH$ libre situé en alpha d'un groupe OH.

8. Utilisation d'un conjugué haptène-protéine préparé selon l'une des revendications 1 à 7 pour l'immunisation d'organismes convenant pour la formation d'anticorps.

9. Utilisation d'un conjugué haptène-protéine préparé selon la revendication 5 ou 6 comme enzyme de marquage dans une détermination immunologique.